Europäisches Patentamt

European Patent Office

Office européen des brevets

(11) Publication number: **0 206 767**
A2

(12) **EUROPEAN PATENT APPLICATION**

(21) Application number: **86304720.5**

(22) Date of filing: **19.06.86**

(51) Int. Cl.⁴: **A 61 B 17/58**

(30) Priority: **22.06.85 GB 8515870**

(43) Date of publication of application: **30.12.86**
**Bulletin 86/52**

(84) Designated Contracting States: **AT BE CH DE FR GB IT LI LU NL SE**

(71) Applicant: **A.W. SHOWELL (SURGICRAFT) LIMITED, Britten Street, Redditch Worcs. B97 6HF (GB)**

(72) Inventor: **McMinn, Derek James Wallace, The Thatch Windmill Hill, Stourlton Nr. Worcester (GB)**

(74) Representative: **Hulse, Thomas Arnold et al, Hulse & Co. Cavendish Buildings West Street, Sheffield S1 1ZZ (GB)**

(54) **Bone fixation plates.**

(57) Fracture healing is beneficially influenced by using a bone fixation plate (10) formed of stainless steel with holes (11) for fixing screws and coated with flexible electrically insulating material (13) at least over one face (14) intended to be placed against the bone, the coating preferably and conveniently also extending over all the other surfaces (16, 17, 18) of the plate, except for those surfaces within the screw holes (11) that will be contacted by fixing screws, which surfaces remain as bare metal.

This invention relates to bone fixation plates and is particularly concerned with the influence of internal fixation on the biology of fracture healing.

When long bones are fractured they heal together initially by bridging periosteal callus. However this process is fairly slow and for example in the human tibia it is usually three months before the limb can be left unprotected. During this time the leg has to be enclosed in plaster to maintain alignment of the broken bones. The plaster however must include the knee joint and foot in order to maintain control of the broken tibia. A complication of this is stiffness of the knee, ankle and foot due to the long period of immobilisation.

It is now common practice to treat bones like the broken tibia by operation. This involves reduction of the fracture and fixation by a plate and screws. No plaster is necessary, the neighbouring joints can be moved and secondary stiffness is not a problem.

The first plates and screws were flimsy and bending and breaking of the plates was a common occurrence. Today plates and screws are of much better design and this early mechanical failure is not so much of a problem. This rigid internal fixation with metal plates and screws does, however, affect the biology of fracture healing in long bones.

If internal fixation with bone plates and screws is performed perfectly (i.e. with perfect reduction bringing the fractured bone ends into absolutely correct alignment and contact) then no bridging periosteal callus forms; instead, the bone ends slowly 'weld' together, which process - now known as "primary bone union" - is, however, very slow and so the patient is dependent on the internal fixation device for a very long time, the recommended minimum period being eighteen months. If the internal fixation is not perfect this process of primary bone union will not take place. The healing of bone by bridging periosteal callus is much more forgiving. If the ends of the bone are not reduced perfectly or if there are some missing pieces then the bridging callus 'covers up' all these imperfections. It has therefore been the aim of many research workers to develop a system of internal fixation which will give stability to the broken bone ends but still allow this process of bridging periosteal callus to form.

One approach considered that the total elmination of movement between the bone ends by use of stainless steel plates inhibited the formation of periosteal callus, so plates made of carbon fibre and epoxy resin (hereafter called "carbon fibre plates") have been tried and while giving the necessary stability have allowed a little movement, and callus formation has occurred. However, in bench tests there is no detectable difference in the stiffness of equivalent carbon fibre plates and stainless steel plates, so there could well be some other difference between them accounting for their different influence on fracture healing.

One possibility could be electrical differences. Both plates are excellent conductors of electricity. However there is a marked difference in their polarisation characteristics when they are placed in electrolyte tissue fluid or blood. For example if hydrogen was taken as the indifferent electrode in tissue fluid, when a stainless steel plate is used as the other electrode it will record negative on a voltmeter, whereas when a carbon fibre plate is used as the other electrode the carbon fibre plate will record positive on a voltmeter. It is well known that electricity is involved in fracture healing. Experimental research has shown that there are biopotentials which are measurable on the surface of bones. In the normal shaft of a long bone there is a slightly positive biopotential. However, when the shaft of the long bone is fractured, in the region of the fracture a negative biopstential can be recorded.

Some fractures fail to unite in the usual time and this is called "non-union" or "delayed union". It is now common practice to stimulate the bone ends to unite using electricity. A small battery is implanted in the leg and a wire is taken from the negative pole of the battery and attached to a small electrode in the region of the non-union. The positive pole of the battery is attached

0206767

well away from the fracture. Around the electrode in the region of the non-union callus forms and usually the fractured ends of bone join up. Experimentally if the positive pole of the battery is attached to the electrode in the region of the non-union it does not join up. Callus fails to form and, indeed, there may even be some destruction of the bone that is already there.

As negativity is good for fracture healing, why then does a stainless steel plate not encourage callus and why does a carbon fibre plate not discourage callus?

Consider the stainless steel plate first. If stainless steel is attached to the negative pole of a battery and used as the electrode in the fracture region then it would be donating electrons and callus would form. However, when used as a bone plate the stainless steel has the effect of creating a 'biological battery' and becomes an acceptor of electrons, so callus cannot be formed and healthy bone cells may be destroyed by ionic erosion. Conversely, a carbon fibre plate as a positive electrode would be an acceptor of electrons and so callus could not be formed, whereas as part of a 'biological battery' it would donate electrons and callus would be formed.

Indeed, carbon fibre bone plates have proved excessively good at encouraging callus, to such an extent that bones disposed alongside each other and either or both of which have been fractured have become bound together with excess callus, so that the use of carbon fibre bone plates has been stopped - at least until this problem is overcome.

There is, however, another big practical disadvantage with carbon fibre bone plates, which is that they cannot be contoured (i.e., bent by the surgeon) to the shape of the bones, so they can only be used satisfactorily to plate straight bones, and many bones are not straight.

Experimental work has, therefore, been carried out with stainless steel bone plates enclosed in electrically insulating material, with two independent experimental centres using the same insulating material, namely RILSAN (Trade Mark) nylon, one with the object of avoiding short-circuiting of the biopotentials, the other with the object of avoiding adverse polarisation in the fracture region.

However, although a nylon coated stainless steel bone plate can be bent to the shape of a bone and does not inhibit callus formation, it has the disadvantages that it is not practical to coat the fixing screws with nylon, so ordinary stainless steel screws must be used the heads of which do not accurately fit the plastic-coated holes and so tend to rub off particles of nylon from between the screw heads and the holes as the screws are tightened, so that the fixation becomes loose and the particles interfere with the fracture healing, and/or the nylon between the screw heads and the holes in the plate cause the fixation to be not nearly as rigid as with plain stainless steel plate.

The object of the invention is to provide a bone fixation plate that is free from the above disadvantages.

According to the present invention, a bone fixation plate is formed of stainless steel with holes for fixing screws and is coated with flexible electrically insulating material at least over one face intended to be placed against the bone, the surfaces within the screw holes that will be contacted by fixing screws being bare metal.

The remarkable thing about a bone plate in accordance with the invention is that, although a short circuit between fractured bone ends will be caused by stainless steel fixing screws making contact with both the bone ends and the bare metal of the plate within the screw holes, the insulation over the substantial area of the plate facing the bone ensures that the metal of the plate does not create a 'biological battery' which prevents callus being formed.

The absence of coating between the heads of fixing screws and the holes ensures that the fixation can be rigidly secured, particularly after any necessary bending to match the shape of a bone, and that no particles of coating will be formed which could interfere with the fracture healing.

The coating preferably also extends at least over the longitudinal edges of the plate, and conveniently over all the surfaces of the plate, except - of course - those within the screw holes that will be contacted by fixing screws. Thus support rods in a coating process can be fitted through the screw holes and keep them free of insulating material.

The plate may be formed by coating with RILSAN nylon a "Dynamic Compression Plate" which has oval holes for the fixing screws, which are inserted eccentrically with a special guide, so that when the screws are tightened the bone ends are urged towards each other.

A number of embodiments of the invention will now be described, by way of example only, with reference to the accompanying drawings in which:-

Figure 1 is an elevation of the bone contacting face of a bone plate in accordance with the invention;

Figure 2 is an enlarged section on the line II-II of Figure 1;

Figures 3 and 4 correspond to Figure 2 but show two further embodiments of bone plate in accordance with the invention;

Figure 5 is a perspective view of the bone pate shown in section in Figure 4, as seen from the "non-contacting" face;

Figure 6 is an X-ray, after removal of the screws and bone plate of an adult female sheep's fractured tibia after three months with a bone plate as in Figures 4 and 5 secured thereto;

Figure 7 is an X-ray, after removal of the screws and bone plate of another adult female sheep's fractured tibia after three months with an identical but completely bare metal bone plate secured thereto;

Figure 8 is a diagram of a "plated" bone, also indicating the areas of periosteal callus measured in the experiments; and

Figure 9 is a bar diagram showing the statistical results of densiometry tests on slices of "plated" bones compared with slices of control bones.

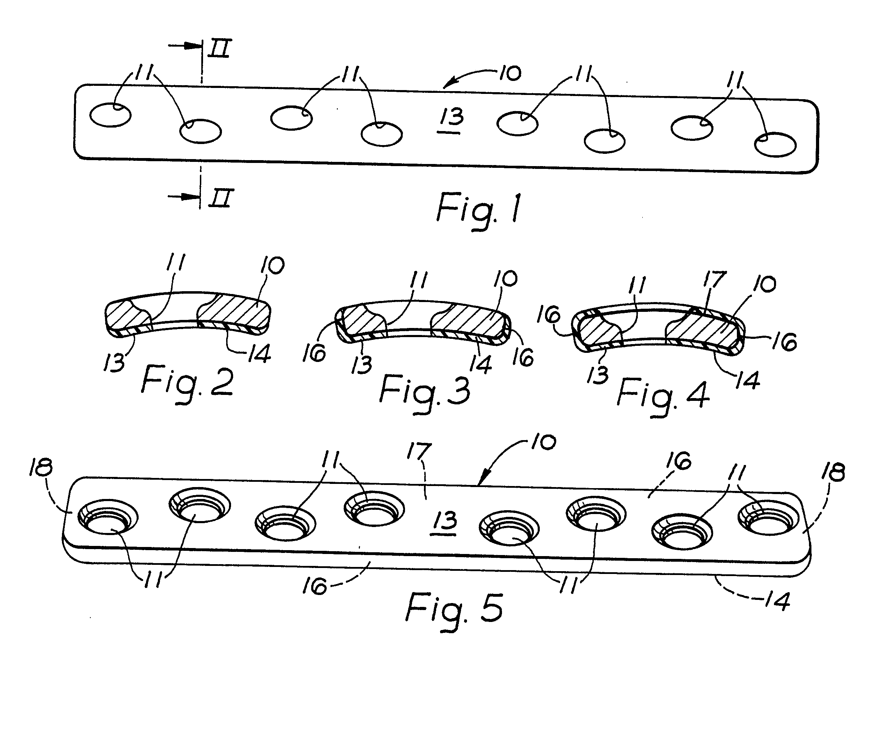

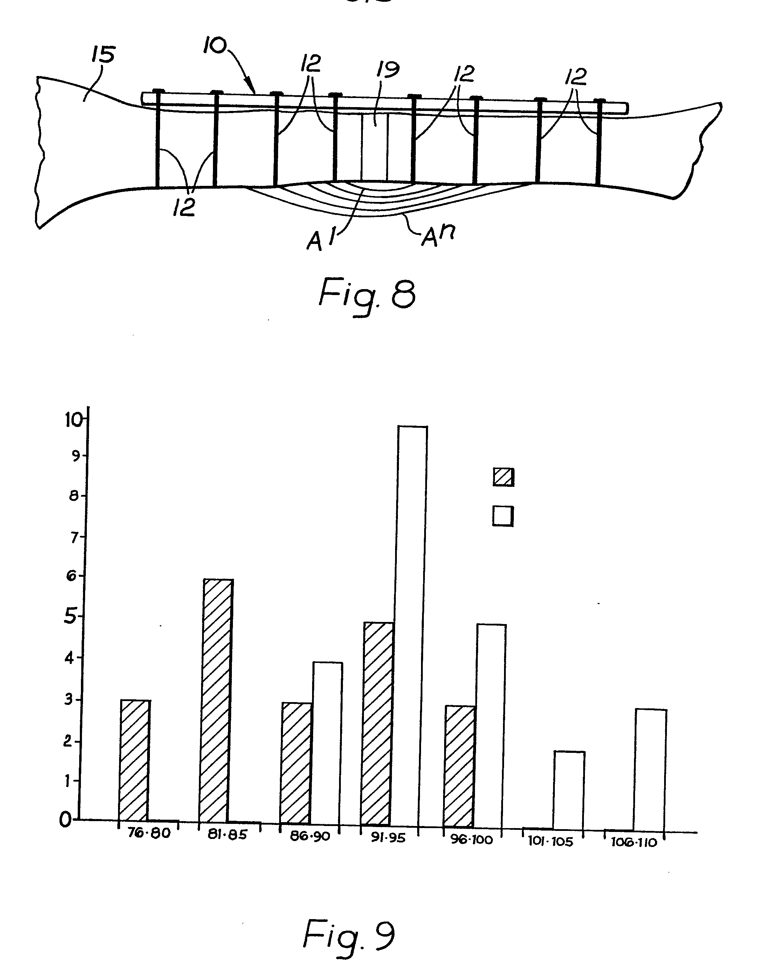

In Figures 1 and 2, a stainless steel bone plate 10 with eight holes 11 for fixing screws (indicated as 12 in the diagram of Figure 8) is coated with flexible electrically insulating material 13 over one face 14 intended to be placed against the bone 15 (Figure 8), and it should be noted particulatly that the surfaces within the screw holes 11 that will be contacted by the fixing screws 12 are bare metal.

In Figure 3, the coating 13 also extends over the longitudinal edges 16 of the plate 10, while in Figures 4 and 5 the coating 13 is provided over all the surfaces of the plate (namely the face 14, the "non-contacting" face 17, the longitudinal edges 16, and the ends 18), except those surfaces within the screw holes 11 that will be contacted by the fixing screws 12. It is a simple matter for support rods (not shown) through the screw holes 11 during the all over coating process to keep the holes free of insulating material 13, such as RILSAN nylon.

It will be seen that the holes 11 are oval (as well as countersunk) so that when the fixing screws 12 are inserted - in known manner - eccentrically with a special guide and tightened, the bone ends are urged towards each other.

Experiments have been carried out on twelve adult female sheep, one group of six using coated plates 10 as in Figures 4 and 5, and another group of six using identical but completely bare metal plates. The right tibia was used as the experimental side in each animal, and the left tibia served as a control. A standard osteotomy was performed in each case using the McKibben method, leaving a 1cm long section 19 of avascular bone.

In the bare metal group, one tibia shattered after four weeks and at post mortem there was no evidence of fracture healing.

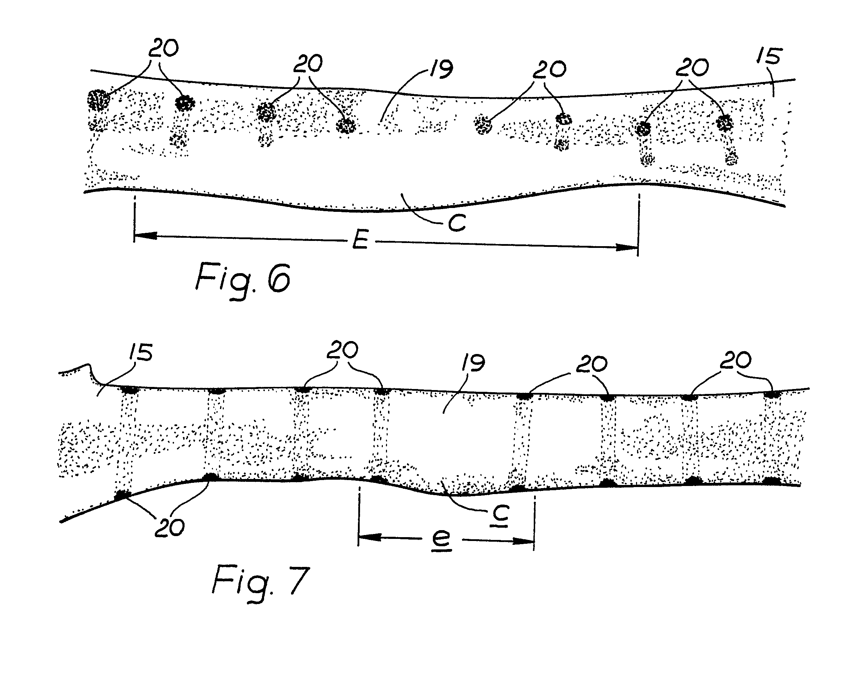

All the other animals were sacrificed at three months and the screws 12 and plates 10 removed. In four out of the five animals in the bare metal group, good healing had taken place but with very little periosteal callus. The best example is shown in the X-ray of Figure 7 in which can be discerned eight holes 20 from which the fixing screws 12 have been removed, as well as the section 19 of avascular bone - even through healing of the bone can be seen to have taken place. The small amount of callus c has a longitudinal extent e.

In the coated group all six tibia held successfully with large amounts of periosteal callus, the best example being shown in the X-ray of Figure 6 in which the excellent healing of the bone is evident from the section 19 of avascular bone being hardly discernable, and the large amount of callus C has a longitudinal extent E.

In Figure 8 the areas of callus are indicated by profile curves $A^1$ and $A^n$, and the areas in the experiments referred to were measured quantitatively by a planimetric technique using Students 't' test (p = .02), the results being as in the following Table 1:-

TABLE 1 - Area of periosteal callus (sq.cm.)

| Bare metal plate | Coated plate |
|---|---|
| 0.0 | 3.5 |
| 0.7 | 1.3 |
| 0.0 | 1.5 |
| 0.2 | 1.7 |
| Non-union | 4.0 |
| Early fracture | 2.0 |
| Mean = 0.225 | Mean = 2.45 |

All the "plated" bones were also tested for strength as compared with the control bones, all bones being subjected to four-point bending to failure using Students 't' test (p = .02), the results being as in the following Table 2:-

TABLE 2 - Strength of "plated" bone as percentage of control bone.

| Bare Metal Plate | Coated Plate |
|---|---|
| 28.5 | 66.0 |
| 42.0 | 55.0 |
| 36.0 | 65.0 |
| 3.6 | 48.0 |
| Non-union | 82.0 |
| Early fracture | Technical failure |
| Mean = 27.53 | Mean = 63.2 |

Slices of bone were cut and microfocal radiographic pictures were taken of all the slices and comparisons made between the "plated" bones and the control bones by densiometry, the results being shown by the bar diagram of Figure 9 and illustrating the significant statistical improvements in densities when using coated plates as compared with bare metal plates.

CLAIMS

1. A bone fixation plate (10) formed of stainless steel with holes (11) for fixing screws (12) and coated with flexible electrically insulating material (13) characterised in that the coating (13) extends at least over one face (14) intended to be placed against the bone (15), and in that the surfaces within the screw holes (11) that will be contacted by fixing screws (12) are bare metal.

2. A bone fixation plate as in Claim 1, characterised in that the coating (13) also extends at least over the longitudinal edges (16) of the plate (10).

3. A bone fixation plate as in Claim 1, characterised in that the coating (13) extends over all the surfaces (14, 16, 17, 18) of the plate (10) except those surfaces within the screw holes (11) that will be contacted by fixing screws.

Fig. 1

Fig. 2

Fig. 3

Fig. 4

Fig. 5

1/3

0206767

Fig. 6

Fig. 7

0206767

Fig. 8

Fig. 9